# EUROPEAN PATENT SPECIFICATION

(11) **EP 0 375 852 B1**
(45) Date of publication and mention of the grant of the patent: **06.09.1995**
(21) Application number: 89118345.1
(22) Date of filing: 03.10.1989
(51) Int. Cl.: A23J 1/20, A61K 35/20, A23C 9/142

(54) **Biologically active whey protein composition, a method for producing it and use of the composition**
Biologisch aktive Molkeproteinzusammenzetzung, Verfahren zur Herstellung und Verwendung dieser Zusammensetzung
Composition de protéines de petit lait biologiquement active, méthode de production et utilisation de la composition

(30) Priority: 23.12.1988 US 289971
(43) Date of publication of application: 04.07.1990
(73) Proprietor: IMMUNOTEC RESEARCH CORPORATION LTD., Montreal, Quebec H3A 2R7 (CA)
(72) Inventor: Bounos, Gustavo, Montreal, Quebec (CA); Gold, Phil, Quebec H3Y 2S4 (CA)
(74) Representative: Körber, Wolfhart, Dr.rer.nat.

(56) References cited:
- EP-A- 0 239 722
- WO-A-87/04050
- WO-A-88/08673
- FR-A- 2 296 428
- GB-A- 1 136 309
- GB-A- 1 495 940
- US-A- 4 710 387
- US-A- 4 753 926
- US-A- 4 784 685
- M.K. Horwitt, The Vitamins, 2nd ed., vol.V, 1972, pages 88-96

## Description

The present invention relates to a biologically active whey protein composition comprising substantive amounts of a whey protein concentrate comprising a whey protein isolate mixture having protein and/or peptide components containing glutamylcysteine groups wherein said components are present in an essentially undenatured state and wherein the biological activity of the whey protein concentrate is restricted to its undenatured conformation. The present invention is also concerned with a method for producing said whey protein composition by ultrafiltration as well as with several application forms of the biologically active whey protein composition as described in claims 13 to 20.

The invention also relates compositions comprising said biologically active whey composition in combination with a neutral carrier (vehicle) suitable for oral feeding.

By oral administration of substantive amounts of undenatured components of cow's whey protein mixture containing glutamylcysteine (Glu-Cys) groups i.e. beta-lactoglobulin and serum albumin and possibly immunoglobulin cellular levels of glutathione (GSH) are increased.

Whey is a well-known dairy industry by-product. Its composition is approximately that of skim milk without its casein. Acid whey is obtained by acidifying the milk either by adding an inorganic acid or by producing lactic acid (seeding the milk with lactic ferments) at a pH near the isoelectric point of the casein. The whey is recovered after separation of the curd. The addition of rennet to the milk also causes the flocculation or coagulation of the casein. The whey obtained after syneresis is called rennet whey. If the flocculation occurs at the pH of milk or at a slightly lower pH but above 5.8 to 6.0, the whey is called sweet whey.

Whey is therefore defined with regard to the nature of the coagulation of the milk. The acid whey results mainly from the fabrication of fresh curds and from casein plants. The composition of the wheys may vary in rather wide ranges depending on the starting milk and the cheese processing employed. All wheys contain mineral, some fats, an amount of lactic acid, coagulant enzymes, the most interesting fraction obviously being the nitrogenous fraction. Indeed, it is the nitrogenous fraction which essentially comprises the soluble milk proteins possessing a high biological value.

The oldest technique for extracting proteins from whey consists of making them insoluble by a denaturing heat treatment at a pH near their isoelectric point. The obvious drawback of such a technique is it denatures the proteins.

As mentioned above, whey proteins are the group of milk proteins that remain soluble in "milk serum" or whey after precipitation of caseins at pH 4.6 and 20°C. The major whey proteins in cow's milk are beta-lactoglobulin (β-L), alpha-lactalbumin (α-L), immunoglobulin and serum albumin (SA) in order of decreasing amounts.

Whey and whey protein have been utilized from time immemorable for nutritional purposes. In addition, whey was recommended in folk and ancient medicine for the treatment of various diseases and, in one instance, lifetime feeding of hamsters with a whey protein diet has been shown to promote longevity with no explanation given.

All these conditions appear to be somehow related to changes in glutathione which is a ubiquitous element exerting a protective effect against superoxide radicals and other toxic agents.

Glutathione is a ubiquitous tripeptide thiol (L-γ-glutamyl-L-cysteinylglycine) with a broad range of vital functions that include detoxification of xenobiotics and protection of cells against oxygen intermediates and free radicals, by-products of oxygen-requiring metabolism. Modulation of intracellular glutathione affects the proliferative immune response of lymphocytes which may be inhibited by oxidative injury. Glutathione protects the cells against radiation and alkylating agents. Age-related or experimentally induced glutathione depletion in the lens is associated with cataract formation. Oxidative DNA damage is rapidly and effectively repaired. The human body is continually repairing oxidized DNA. A small fraction of unrepaired lesions, however, could cause permanent changes in DNA and might be a major contributor to old age diseases and cancer. Indeed, several age associated diseases may be induced by free radicals. It appears that whereas data on age-related changes in tissue vitamin E and other antioxidants are, at best, contradictory, the tissue glutathione levels are more consistently reported to decline with old age in laboratory animals and man.

For these reasons there has been interest in the factors that influence intracellular glutathione synthesis and especially in ways of increasing cellular levels of glutathione.

Glutathione is composed of three amino acids: glutamic acid, glycine and cysteine. Availability of cysteine is a limiting factor in the synthesis of glutathione. Cysteine is derived from dietary protein and by trans-sulfuration from methionine in the liver. Various methods have been tried in order to increase cellular level of glutathione. Administration of free cysteine is not an ideal method because this amino acid is rapidly oxidized, toxic and may actually cause glutathione depletion. Similar problems have been encountered with i.p. injection of N-acetyl-cysteine to rats, although oral administration of this compound appeared to prevent paracetamol-induced glutathione depletion. Administration of compounds that are transported and converted intracellularly into cysteine, such as L-2-oxothiazolidine-4-carboxylate are useful in increasing cellular glutathione acting as an intra-cellular delivery system for cysteine. Hepatic glutathione doubled four hous after injection, returned to normal 8 hours later but was below normal after 16 hours. Another approach for increasing tissue glutathione levels was found in s.c. injection of γ-glutamylcyst(e)ine in mice: glutathione increased in the kidney by about 55%, 40-60 minutes after injection, returning to near control values 2 hours later. The administered compound is transported intact and serves as a substrate for glutathione synthetase. It was also reported that about 2 hours after i.p. administration of γ-glutamyl-cysteinyl-glycyl-monomethyl (or monoethyl) ester to mice, the liver and kidney glutathione levels were doubled, with return to normal values after 8 hours. Similar increases in glutathione tissue levels were attained by Meister by administering an alkyl monoester of glutathione (US-A-4,784,685) to mice. Such esters are transported into tissue cells, and are de-esterified within the cells, thus leading to increased cellular levels of glutathione. The kinetics of tissue glutathione increments attained with this method are similar to those described following i.p. injection of methyl or ethyl esters of glutathione. The effectiveness of these methods has been clearly demonstrated in acute experiments (US-A-4,784,685); in mice treated with L-2-oxothiazolidine-4-carboxylate the expected drop in glutathione tissue level subsequent to acetaminophen injection, was replaced by an actual increase in tissue glutathione values and survival. Other methods to increase tissue glutathione levels are based on the "overshoot" of glutathione concentration, following depletion by diethylmaleate or BSO. These studies were done in vitro on murine cell lines. Also preexposure of rats to hypoxia was found to increase lung glutathione.

The administration of glutathione itself is of little consequence on tissue glutathione levels, because it apparently cannot be transported intact across the cell membrane.

Some of the previously discussed methods of increasing intracellular levels of glutathione concentration are either toxic or dangerous owing to the risks related to the initial phase of glutathione depletion. The methods involving the use of γ-glutamylcyst(e)ine, athiazolidine or glutathione esters (US-A-4,784,685) offer an interesting possibility for short term intervention. However, their long term effectiveness in producing sustained elevation of cellular glutathione has not been shown, nor has the possible toxicity of their long term use been disproved. Indeed, glutathione and glutathione disulfide were found to be positive in the most commonly used short term tests for carcinogenicity and mutagenicity.

Accordingly, an object of the invention is to provide a novel whey protein composition which is biologically active and which can be used in the nutrition of human and animals and as drugs, the composition should contain a special whey protein fraction or fractions which increase host cellular glutathione, reducing the total amount required in comparison to that what is needed to obtain similar objectives using undenatured whey protein concentrate, the new product should be administrated either in capsule or as a powder to be easily dissolved in water or other liquids.

Another object of the invention is to provide a way to increase the intracellular levels of glutathione by oral administration of the cysteine precursor as contained in the undenatured whey protein concentrate of bovine origin. Similar amounts of cysteine given as free cysteine are ineffective. On the other hand, the same amounts of another protein such as egg white, with an identical high cysteine content, were found to be ineffective in raising tissue glutathione levels. Hence, the specific whey protein concentrate physico-chemical composition, in its undenatured form (i.e. the position of cysteine in relation to the primary, secondary and tertiary structure of the protein), appears to be a crucial factor in the bioavailability of cysteine as a precursor for intracellular synthesis of glutathione.

Another object of the invention is to provide a method for increasing cellular levels of glutathione without exposure to the toxic effects associated with other known methods: the long term ingestion of bovine whey protein concentrate has been shown to be non-toxic in mice, hamsters and humans.

A further object of the invention is to provide a sustained elevation of tissue glutathione for any purpose for which a prophylactic long term elevation of cellular glutathione levels is desired in the prior art, such as for cellular protection against free radicals, foreign hazardous compounds, drug detoxification, radiation, immunodeficiency states etc.

It was surprisingly found upon technical changes in the industrial methods of preparation of whey from milk and of whey protein concentrate (w.p.c.) that w.p.c. must be undenatured in order to be biologically active.

Accordingly, a new concept relating to the preparation of whey protein concentrate, with the specific aim of preserving the undenatured conformation upon which its newly discovered biological activity is dependant is proposed in the present invention. This naturally involves the two principal phases in the industrial production of whey proteins:
1. A process of separation of whey from milk which brings about minimal deviation of the whey protein conformation from the range normally accepted as native. Hence the process should exclude levels of heat treatment and shaking in the presence of air which could denature the whey protein.
2. A process for whey protein concentrate production designed to recover from whey the proteins in their native conformation. Hence ultra-filtration and the other stages of the process are very lenient excluding or limiting heating, pumping, aeration and other processing treatment which promotes protein denaturation.

The above-mentioned problems were solved by the characterizing features of claim 1.

The invention is further developed by the subclaims.

Claims 13 to 20 comprise several application forms of the novel whey protein composition.

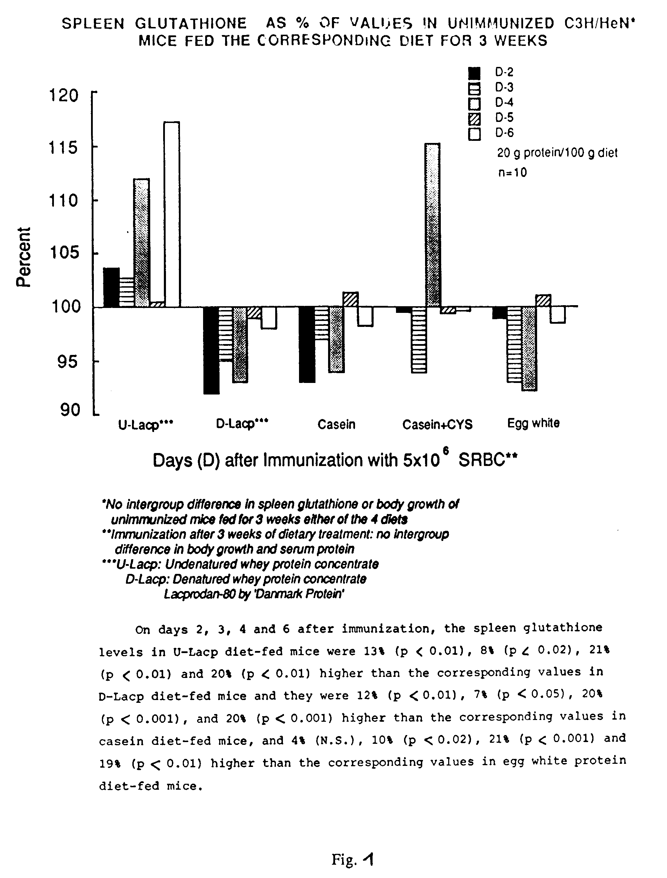

The advantages offered by the invention are mainly that:
1. Oral administration of substantive amounts of bovine whey protein concentrate enhances the glutathione content in the liver, heart and spleen of mice (see Fig. 1, 2, 3).
2. This change is moderate but sustained over time and biologically significant.
3. This property is restricted to the undenatured conformation of whey protein concentrate.
4. Glutathione is a tripeptide (L-γ-glutamyl-L-cysteinyl-glycine).
   The administration of either cysteine (limiting substrate) or glutathione itself or other protein sources such as egg white are ineffective in raising cellular levels of GSH. However, i.p. injection of γ-glutamyl-cysteine was found to enhance for a few hours the tissue GSH level of mice.
5. Whey protein concentrate contains substantial amounts of glutamylcysteine groups, unlike casein, which does not increase tissue GSH when fed to mice.
6. The glutamylcysteine groups are located in the beta-lactoglobulin and serum albumin fractions (see table 1). The aminoacid sequence of bovine milk immunoglobulin is not totally known.
7. Pancreatic digestion does not split the disulfide bond of the whey proteins; instead heat denaturation is known to split this bond, thus unfolding the globular structure of the molecule to form a random coil conformation.

The invention is now described with reference to the drawings and tables which show:
- Fig. 1: the spleen glutathione content of mice fed different diets,
- Fig. 2 and 3: liver and heart glutathione content of mice fed different diets,
- Fig. 4: enhancement of spleen cell immune response to SRBC, in mice fed a diet containing undenatured whey protein concentrate,
- Fig. 5: role of glutathione in the immuno-enhancing effect of dietary whey protein,

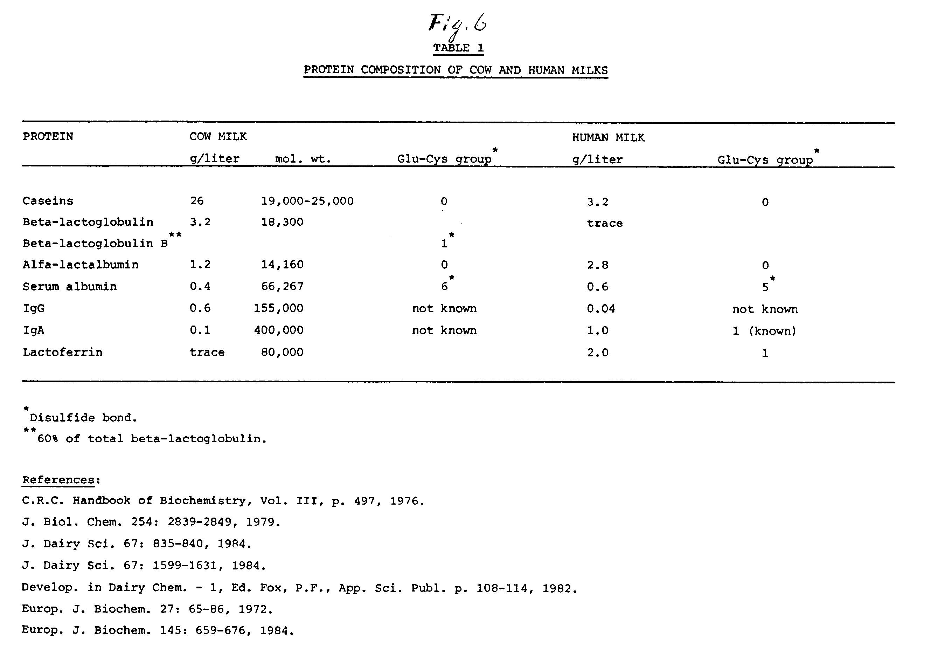

- Fig. 6 Table 1: the protein composition of cow and human milks,

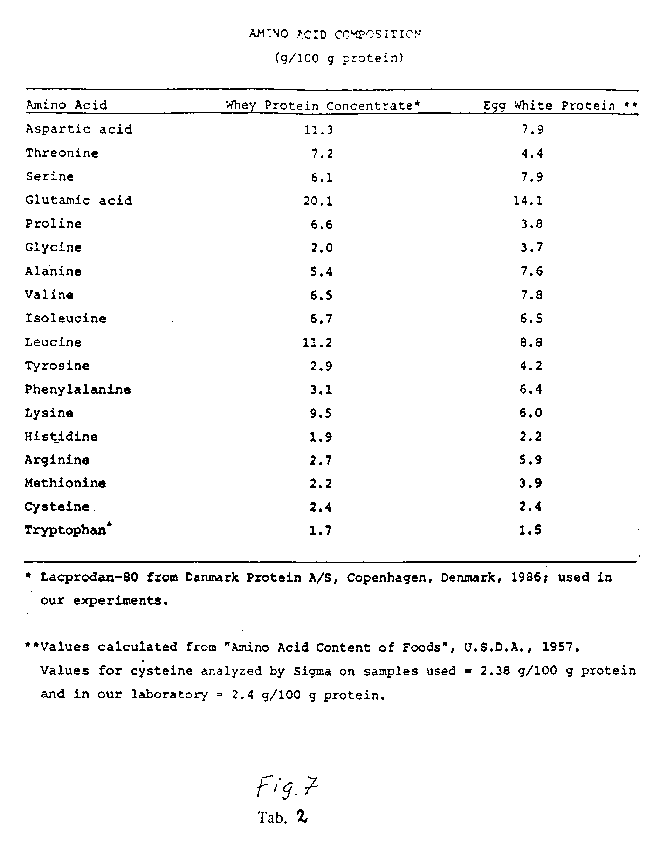

- Fig. 7 Table 2: amino acid composition of whey protein concentrate and egg white protein.

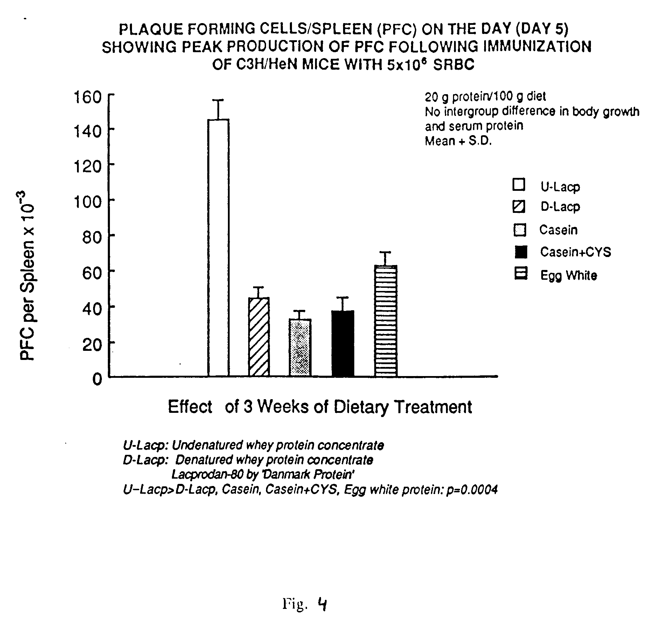

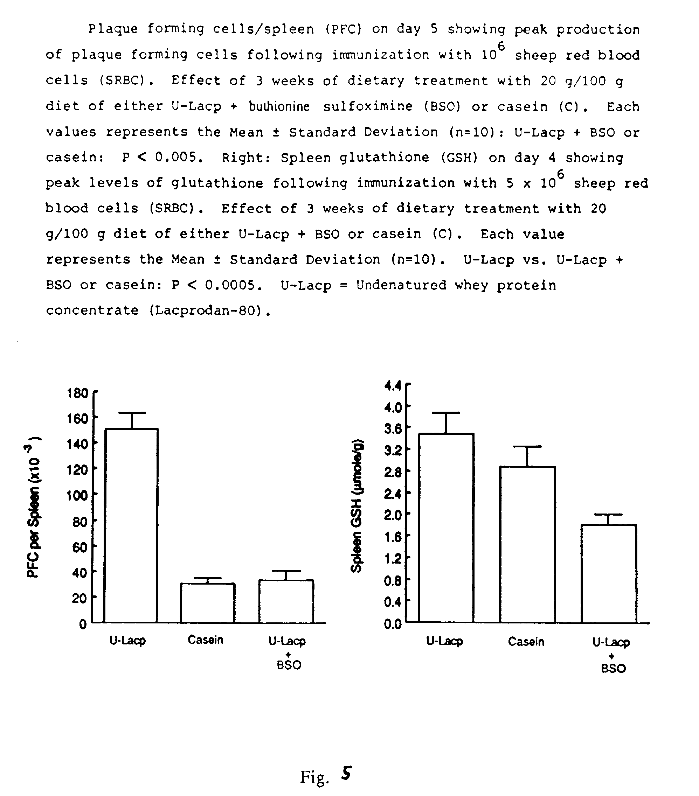

The interaction of dietary protein, GSH and the host immune response was explored. It was investigated whether a different protein source such as egg white, with the same high level of cysteine as whey protein concentrate (Table 2), had a similar effect in promoting higher GSH tissue content. It was found out that an egg white protein diet does not enhance the host immune response above average. Whereas the static GSH level in spleen was found unaltered by U-Lacp feeding for three weeks, the present studies in young adult C3H mice showed that enhancement of spleen cell immune response to SRBC (Fig. 4) is associated with sustained elevation of splenic GSH during the antigen-driven clonal expansion of the lymphocytes in U-Lacp (undenatured whey protein)-fed mice in comparison to a pattern of decline observed in spleen GSH levels in mice fed either of the nutritionally equivalent D-Lacp (denatured whey protein), casein, cysteine enriched casein, or egg white protein diets (Fig. 1). The latter four groups also exhibited a lower immune response (Fig. 4). Administration of S-(n-butyl) homocyteine sulfoximine, which reduces the splenic glutathione level by half, produces a marked drop in the humoral immune response of whey protein (U-Lacp) diet-fed mice. This is further evidence of the important role of glutathione in the immunoenhancing effect of dietary whey protein (Fig. 5).

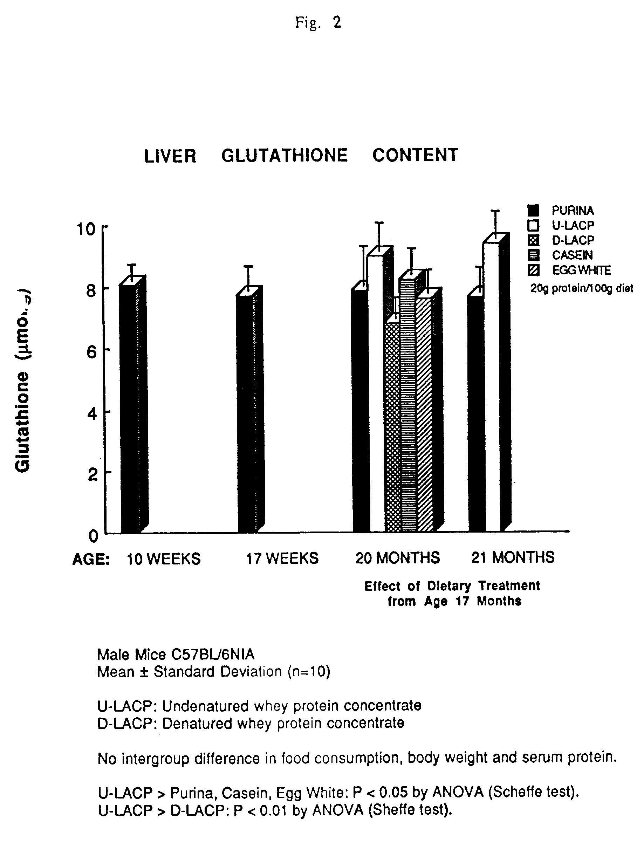

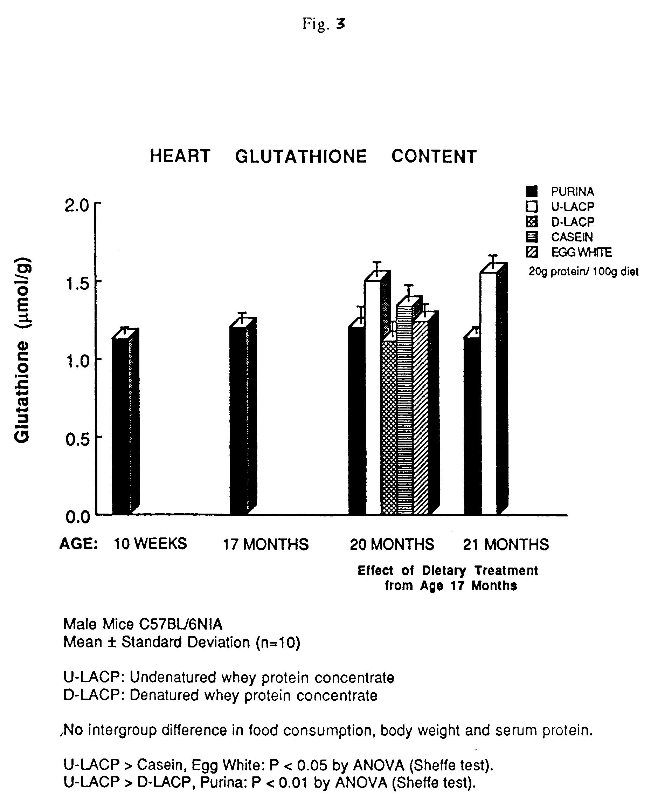

Tissue Glutathione Assay: Ninety milligrams of mouse heart or liver were homogenized in 5-sulfosalicylic acid (5% w/v). Homogenates are centrifuged for 5 minutes in a microfuge at 10,000 x g. The assay is carried out using the supernatants on the same day according to the method of Anderson. Values are expressed as µmol/g wet tissue (Fig. 2 and 3).

After three months on either diet initiated at age 17 - months, GSH content was found to be higher in the liver and heart of U-Lacp (undenatured whey protein) fed mice compared to the D-Lacp (denatured whey protein), casein, egg white protein or Purina diet-fed counterparts (Fig. 2 and 3). The GSH values in heart and liver of mice fed Purina laboratory chow was similar at age 10 weeks, 17, 20, 21 months. The U-Lacp diet appears to enhance the GSH content of heart and liver above "normal" values after 3 and 4 months of continuous feeding (Fig. 2 and 3).

In conclusion, after three weeks on the U-Lacp diet, spleen GSH content is increased during the antigen driven clonal expansion of the lymphocytes in young adult C3H/HeN mice as compared to a decline in controls fed D-Lacp, casein or egg white protein diets (Fig. 1). In old C57BL/6N1A mice, long term feeding of U-Lacp diet results in a moderate but sustained increase in liver and heart GSH levels (Fig. 2 and 3). The GSH enhancing activity of WPC (whey protein concentrate) is restricted to its undenatured form (U-Lacp). This property is not solely due to the high cysteine content of WPC because another protein source with similar cysteine content (egg white) does not exhibit this biological activity. This property of U-Lacp does not depent specifically on its nutritional efficiency as evaluated by body weight, serum proteins, and food consumption, but appears to depend on the primary, secondary and tertiary structure of the protein in its native form.

Data in Fig. 2 and 3 show that the concentration of liver and heart glutathione in control Purina fed mice remains very constant over time. On the other hand a moderate but sustained elevation of tissue GSH was noted in mice fed the nutritionally equivalent undenatured whey protein (U-Lacp) diet. Only minuscule quantities of glutathione and no breakdown products that can be readily attributed to glutathione are excreted in urine. The magnitude of change in cellular glutathione concentration that can be achieved may be quite limited, perhaps reflecting the critical importance of this molecule and the attendent tight regulatory control. Glutathione itself serves as a negative feedback on the GSH synthetic enzymes, which obviously limits cellular capacity to increase GSH concentration. Glutathione reductase maintains GSH in its predominant reduced form (> 90%). This serves both to maintain this functional state and also to control cellular concentration since reduced glutathione (GSH) cannot cross the membrane, whereas the oxidized form (GSSG) can and does efflux, resulting in decreased total glutathione. Besides these enzymes, gamma glutamyltranspeptidase (GGT) is important in GSH metabolism. GGT serves as a salvage pathway for glutamyl moieties at the cell membrane level, passing them back into the cytosol to be used in GSH synthesis. Increased activity of this enzyme has been associated with elevated GSH concentration in a number of cell lines and malignant tissues.

The effects of a small increment in cellular GSH may be greater than expected. For example, there are many reports of human and murine tumor cell lines selected in vitro for resistance to a variety of chemotherapeutic agents. In a number of these cell lines cellular GSH is increased consistently by 2-fold compared to the drug sensitive parental cell line, despite the fact that the level of drug resistance is often much greater, e.g. as much as 30-fold. In these cell lines, depletion of cellular GSH by selective inhibition of synthesis restores drug sensitivity to the resistent cells. This is effective only if the GSH depletion is maintained throughout the drug-treatment period.

Given the fact that cellular GSH is very tightly regulated, that a 2-fold increase may be maximal, and that the effect of small increments in GSH may be amplified by a variety of GSH-utilizing enzymes (e.g. glutathione peroxidase, glutathione-S-transferase), the reproducible change in GSH concentration observed in animals fed the whey-rich diet is likely to have biological importance. The chronic nature of this augmentation may contribute significantly to this effect.

Relevant to the present invention are recent data indicating specifically that a lack of the GSH precursor, cysteine, rather than a decrease in biosynthetic enzyme activities is responsible for the deficiency of GSH noted in aging animals. Similarly, the fall in cytosolic GSH in the liver of chronic ethanol fed rats does not appear to be caused by a limitation in the capacity of γ-glutamyl-cysteine synthetase activity.

### Summary and Signification of the Invention

a. The glutathione promoting activity of dietary whey protein concentrate is dependant on the glutamylcysteine groups contained in the beta-lactoglobulin and serum albumin fractions and possibly in the IgG fraction.
b. The preservation of the disulfide bond (which involves the cysteine) may be crucial to the release, upon digestion, of intact glutamylcysteine peptide for absorption by the intestinal mucosa. Denaturation, on the other hand, by unfolding the protein molecule, exposes the glutamylcysteine sequence to the digestive enzymes with subsequent release of either of the single aminoacids or other peptide combinations. This would be consistent with the observed absence of glutathione promoting activity in the denatured form of whey protein concentrate.

The present invention provides a method to remove from whey protein the alpha-lactalbumin (table 1).

Oral administration of the whey protein fraction or fractions found to release most glutamylcysteine groups upon digestion, with the purpose of increasing host cellular glutathione. The total amount required would be significantly less than what is needed to obtain similar objectives using undenatured whey protein concentrate. Hence the product could be administered either in capsule or as a powder to be easily (undenatured) dissolved in water or other liquids.

## Claims

1. Method of producing a biologically active whey protein concentrate containing a suitable concentration of undenatured whey protein being free of α-lactalbumin comprising the following steps:
a) immediately after milking, cooling the milk to a temperature in the range of 2°C to 10°C, especially to 4°C, and removing contaminants,
b) after another cleaning of the milk, precipitation of the curd by reducing the pH to about 4.6 with lactic acid initially at 20°C,
c) addition of rennet and raising the temperature to about 30°C for 20 minutes to promote expulsion of whey from the curd, allowing the agitation in a vat to resolve at low speed,
d) irradiation and separation of the whey, and
e) ultrafiltration of the whey using a membrane having a molecular weight cut off of substantially 17,000 to retain β-lactoglobulin and serum albumin,
characterized in that the fraction of whey protein is not heated and the material from which it is derived is slowly agitated to minimize protein denaturation and that said ultrafiltration is carried out in a production line comprising up to 20 frame-type modules holding a large number of said membranes achieving from the retentate a final undenatured α-lactalbumin-free protein concentrate in dry matter, wherein said ultrafiltration is carried out at a temperature in the range of 4°C to 20°C, especially at 4°C.

2. Method of producing an undenatured whey protein concentrate according to claim 1, characterized in that the whey protein concentrate is after ultrafiltration free of lactose, salts and water.

3. A method according to any one of the preceding claims, wherein the temperature of ultrafiltration and/or the temperature of cooling in step a) is 4°C.

4. A biologically active substantially undenatured whey protein concentrate enriched in the glutamylcysteine groups primarily contained in the serum albumin and betalactoglobulin fractions of the whey protein and which may also contain immunoglobulin.

5. A biologically active whey protein concentrate as in claim 4 in which the concentrate has been enriched by the substantial removal of the α-lactalbumin fraction.

6. Whey protein concentrate according to claim 4 or 5, wherein the whey protein isolate mixture is further concentrated and refined to medicinal purity.

7. Whey protein concentrate according to claims 4 to 6, wherein the whey protein concentrate has immunoenhancing proteins being heat-labile and insensitive to pancreatic digestion and wherein the immunoenhancing properties depend upon the undenatured state of the peptides and proteins contained in said whey protein mixture.

8. Whey protein concentrate according to claim 4, wherein the whey protein concentrate is one selected from the group consisting of bovine and/or goat and/or sheep and/or human whey protein concentrate.

9. Whey protein concentrate according to claim 4 or 5, wherein said whey protein concentrate is for administration of a therapeutically or prophylactically effective amount of 18 to 28g whey protein/100g diet, especially 20g whey protein/100g diet.

10. Whey protein concentrate according to any one of the preceding claims comprising in combination said whey protein concentrate together with Vitamins B₁ and B₂ in amounts for administration in excess of minimum daily requirements.

11. Whey protein concentrate according to claim 10 wherein the vitamin amounts are 1.5 to 2.0 mg B₁ and 1.5 to 2.0 mg B₂.

12. Use of the whey protein concentrate according to any one of claims 4 to 11 in a concentration of 18 to 28g whey protein/100g diet for the manufacture of a food product as a substitute for milk or milk protein.

13. Use of the whey protein concentrate according to claims 4 to 12 for the manufacture of a food product as a food substitute for avoiding the tendency of lactose malabsorption.

14. Use of the whey protein concentrate according to claims 4 to 11 for the manufacture of a food product as substitutes or complements for dairy products, for human or animal alimentation.

15. Use of a whey protein concentrate according to any one of claims 4 to 11 in the manufacture of a therapeutic composition.

16. Use of a whey protein concentrate according to claim 15 in the manufacture of a drug for increasing the rate of synthesis, rate of replenishment, and/or concentration levels of glutathione in human and animal organs, in a therapeutically or prophylactically effective amount of the whey protein concentrate.

17. Use of a whey protein concentrate according to claim 15 in the manufacture of a drug for improving host resistance in humans and animals in a therapeutically or prophylactically effective amount of the whey protein concentrate.

18. Use of the whey protein concentrate according to claim 17, wherein the improved host resistance comprises enhanced resistance to bacterial infection and/or enhanced resistance to slow growing carcinoma and/or enhanced resistance to the process of aging and/or a combination of the above.

19. Use of a whey protein concentrate according to claim 15 in the manufacture of an anti-cancer therapeutic composition intended for the prophylaxis of colon cancer.

## Patentansprüche

1. Verfahren zur Herstellung eines biologisch wirksamen Molkeproteinkonzentrats, das eine geeignete Konzentration an nicht-denaturiertem, α-Lactalbumin-freien Molkeprotein enthält, umfassend die folgenden Schritte:
a) sofort nach dem Melken, Kühlen der Milch auf eine Temperatur zwischen 2°C und 10°C, besonders 4°C, und Entfernen der Verunreinigungen,
b) nach weiterem Reinigen der Milch, Fällen der geronnenen Milch anfänglich bei 20°C durch Verringern der pH-Werts mit Milchsäure auf etwa 4,6,
c) Zugabe von Lab und 20 minütiges Erhöhen der Temperatur auf etwa 30°C, um die Austreibung von Molke aus der geronnenen Milch zu beschleunigen, wobei zur Trennung in einem Bottich bei niedriger Geschwindigkeit gerührt wird,
d) Bestrahlen und Abtrennen der Molke, und
e) Ultrafiltration der Molke unter Verwendung einer Membran mit einer Molekularmassen-Abtrennung von im wesentlichen 17000, um β-Lactalbumin und Serumalbumin zurückzuhalten,
dadurch gekennzeichnet, daß der Anteil des Molkeproteins nicht erhitzt wird und das Material, aus dem es erhalten wird, langsam gerührt wird, um Proteindenaturierung zu minimieren und daß die Ultrafiltration in einer Fertigungsstraße durchgeführt wird, die bis zu 20 Rahmenähnliche, eine Vielzahl der Membrane tragende Module enthält, wobei aus dem Rückstand ein endgültiges nichtdenaturiertes α-Lactalbumin-freies Protein-Konzentrat in Trockenform erhalten wird, worin die Ultrafiltration bei einer Temperatur von 4°C bis 20°C, besonders 4°C, durchgeführt wird.

2. Verfahren zur Herstellung eines nicht-denaturierten Molkeproteinkonzentrats gemäß Anspruch 1, dadurch gekennzeichnet, daß das Molkeproteinkonzentrat nach Ultrafiltration frei von Lactose, Salzen und Wasser ist.

3. Verfahren gemäß einem der vorstehenden Ansprüche, worin die Temperatur der Ultrafiltration und/oder die Kühltemperatur in Schritt a) 4°C ist.

4. Biologisch wirksames, im wesentlichen nichtdenaturiertes Molkeproteinkonzentrat, das an Glutamylcysteinresten angereichert ist, die vorwiegend im Serumalbumin und in Betalactoglobulin-Fraktionen des Molkeproteins enthalten sind und die ebenfalls Immunglobulin enthalten können.

5. Biologisch wirksames Molkeproteinkonzentrat wie in Anspruch 4, in welchem das Konzentrat durch die wesentliche Entfernung der α-Lactalbumin-Fraktion angereichert wurde.

6. Molkeproteinkonzentrat gemäß Anspruch 4 oder 5, worin das isolierte Molkeprotein-Gemisch weiter konzentriert und auf medizinische Reinheit gereinigt ist.

7. Molkeproteinkonzentrat gemäß der Ansprüche 4 bis 6, worin das Molkeproteinkonzentrat immunsteigernde, hitzelabile und gegen pankreatische Verdauung unempfindliche Proteine hat und worin die immunsteigernden Eigenschaften vom nicht-denaturierten Zustand der Peptide und Proteine, die in dem Molkeproteingemisch enthalten sind, abhängen.

8. Molkeproteinkonzentrat gemäß Anspruch 4, worin das Molkeproteinkonzentrat aus Rinder- und/oder Ziegen- und/oder Schaf und/oder menschlichem Molkeproteinkonzentrat ausgewählt wird.

9. Molkeproteinkonzentrat gemäß Anspruch 4 oder 5, worin das Molkeproteinkonzentrat zur Verabreichung einer therapeutisch oder prophylaktisch wirksamen Menge von 18 bis 28 g Molkeprotein/100 g Diät, besonders 20 g Molkeprotein/100 g Diät, ist.

10. Molkeproteinkonzentrat gemäß einem der vorstehenden Ansprüche, umfassend in Verbindung das Molkeproteinkonzentrat mit Vitaminen B₁ und B₂ in Mengen zur Verabreichung im Überschuß eines minimalen täglichen Bedarfs.

11. Molkeproteinkonzentrat gemäß Anspruch 10, worin die Vitaminmengen 1,5 bis 2,0 mg B₁ und 1,5 bis 2,0 mg B₂ sind.

12. Verwendung des Molkeproteinkonzentrats gemäß einem der Ansprüche 4 bis 11 in einer Konzentration von 18 bis 28 g Molkeprotein/100 g Diät zur Herstellung eines Nahrungsmittels zum Ersatz von Milch oder Milchprotein.

13. Verwendung des Molkeproteinkonzentrats gemäß einem den Ansprüchen 4 bis 12 zur Herstellung eines Nahrungsmittels als eine Ersatznahrung, um die Neigung zur Lactose-Malabsorption zu vermeiden.

14. Verwendung des Molkeproteinkonzentrats gemäß den Ansprüchen 4 bis 11 zur Herstellung eines Nahrungsmittels als Ersatz oder Ergänzung für Milchprodukte, beim Menschen oder in Tiernahrung.

15. Verwendung eines Molkeproteinkonzentrats gemäß einem der Ansprüche 4 bis 11 zur Herstellung eines therapeutischen Mittels.

16. Verwendung eines Molkeproteinkonzentrats gemäß Anspruch 15 bei der Herstellung eines Medikaments zur Erhöhung der Synthesegeschwindingkeit,
Ergänzungsgeschwindigkeit, und/oder Konzentrationsspiegeln von Glutathion in menschlichen oder tierischen Organen, in einer therapeutisch oder prophylaktisch wirksamen Menge des Molkeproteinkonzentrats.

17. Verwendung eines Molkeproteinkonzentrats gemäß Anspruch 15 bei der Herstellung eines Medikaments zur Verbesserung der Wirstsbeständigkeit beim Menschen und in Tieren in einer therapeutisch oder prophylaktisch wirksamen Menge des Molkeproteinkonzentrats.

18. Verwendung des Molkeproteinkonzentrats gemäß Anspruch 17, worin die verbesserte Wirtsbeständigkeit erhöhte Wiederstandskraft gegen bakterielle Infektionen und/oder erhöhte Widerstandskraft gegen langsam wachsende Karzinome und/oder erhöhte Widerstandskraft gegen den Prozess des Alterns un d/oder eine Kombination des Vorstehenden umfaßt.

19. Verwendung eines Molkeproteinkonzentrats gemäß Anspruch 15 zur Herstellung einer therapeutischen Antikrebszusammensetzung, welche zur Vorbeugung gegen Dickdarmkrebs beabsichtigt ist.

## Revendications

1. Procédé de production d'un concentré de protéines de lactosérum, actif biologiquement, contenant une concentration adaptée de protéines de lactosérum non-détanurée exempte d'α-lactalbumine, comportant les étapes suivantes :
a) immédiatement après la traite, refroidir le lait à une température située dans la plage allant de 2°C à 10°C, en particulier à 4°C, et enlever les contaminants,
b) après un autre nettoyage du lait, faire précipiter le caillé en réduisant le pH à environ 4,6 à l'aide d'acide lactique initialement à 20°C,
c) ajouter de la présure et élever la température à environ 30°C pendant 20 minutes pour favoriser l'expulsion de lactosérum à partir du caillé, en prévoyant l'agitation dans une cuve pour séparer à faible vitesse,
d) irradier et séparer le lactosérum, et
e) effectuer une ultrafiltration du lactosérum en utilisant une membrane ayant un pouvoir de coupure en poids moléculaire d'à peu près 17 000 pour retenir la β-lactoglobuline et l'albumine du sérum,
caractérisé en ce que la fraction de protéine du lactosérum n'est pas chauffée et la matière à partir de laquelle elle est dérivée est agitée lentement pour minimiser la dénaturation de la protéine et en ce que ladite ultrafiltration est effectuée dans une ligne de production comportant jusqu'à 20 modules du type cadre supportant un nombre important desdites membranes, produisant à partir de la rétention un concentré final de protéines non dénaturées exemptes d'α-lactalbumine, en matière sèche, dans lequel ladite ultrafiltration est effectuée à une température située dans la plage allant de 4°C à 20°C, en particulier à 4°C.

2. Procédé de production d'un concentré de protéines de lactosérum non-dénaturé selon la revendication 1, caractérisé en ce que le concentré de protéines de lactosérum est, après ultrafiltration, exempt de lactose, de sels et d'eau.

3. Procédé selon l'une quelconque des revendications précédentes, dans lequel la température de l'ultrafiltration et/ou la température de refroidissement de l'étape a) est de 4°C.

4. Concentré de protéines de lactosérum pratiquement non dénaturé, biologiquement actif, enrichi de groupes de glutamylcystéine contenus principalement dans les fractions albumine du sérum et β-lactoglobuline de la protéine du lactosérum et qui peut aussi contenir de l'immunoglobuline.

5. Concentré de protéines de lactosérum biologiquement actif selon la revendication 4, dans lequel le concentré a été enrichi par la suppression de pratiquement la fraction α-lactalbumine.

6. Concentré de protéines de lactosérum selon la revendication 4 ou 5, dans lequel le mélange d'isolats de protéines de lactosérum est en outre concentré et raffiné jusqu'à la pureté médicinale.

7. Concentré de protéines de lactosérum selon l'une des revendications 4 à 6, dans lequel le concentré de protéines de lactosérum a des protéines renforçant l'immunité, sensibles à la chaleur et insensibles à la digestion pancréatique et dans lequel les propriétés renforçant l'immunité dépendent de l'état non dénaturé des peptides et protéines contenues dans ledit mélange de protéines de lactosérum.

8. Concentré de protéines de lactosérum selon la revendication 4, dans lequel le concentré de protéines de lactosérum est un élément choisi dans le groupe constitué par des concentrés de protéines de lactosérum bovin, et/ou caprin, et/ou ovin, et/ou humain.

9. Concentré de protéines de lactosérum selon la revendication 4 ou 5, dans lequel ledit concentré de protéines de lactosérum est destiné à l'administration d'une quantité thérapeutiquement ou prophylactiquement efficace de 18 à 28 g de protéines de lactosérum/100 g d'aliments, en particulier de 20 g de protéines de lactosérum/100 g d'aliments.

10. Concentré de protéines de lactosérum selon l'une quelconque des revendication précédentes, comportant en combinaison ledit concentré de protéines de lactosérum associé aux vitamines B₁ et B₂ selon des quantités destinées à une administration dépassant les besoins quotidiens minima.

11. Concentré de protéines de lactosérum selon la revendication 10, dans lequel les quantités de vitamines sont de 1,5 à 2,0 mg pour B₁ et de 1,5 à 2,0 mg pour B₂.

12. Utilisation de concentré de protéines de lactosérum selon l'une quelconque des revendications 4 à 11, selon une concentration de 18 à 28 g de protéines de lactosérum/100 g d'aliments, pour la fabrication d'un produit alimentaire en tant que substitut du lait ou de la protéine du lait.

13. Utilisation du concentré de protéines du lactosérum selon l'une des revendications 4 à 12 pour la fabrication d'un produit alimentaire en tant que substitut d'aliment pour éviter la tendance à une mauvaise absorption du lactose.

14. Utilisation du concentré de protéines du lactosérum selon l'une quelconque des revendications 4 à 11 pour la fabrication d'un produit alimentaire en tant que substitut ou complément de produits laitiers, destiné à l'alimentation humaine ou à l'alimentation animale.

15. Utilisation d'un concentré de protéines de lactosérum selon l'une quelconque des revendications 4 à 11 pour la fabrication d'une composition thérapeutique.

16. Utilisation d'un concentré de protéines de lactosérum selon la revendication 15 pour la fabrication d'un médicament destiné à accroître la vitesse de synthèse, la vitesse de reconstitution et/ou les niveaux de concentration de glutathione dans les organes humains et animaux, selon une quantité thérapeutiquement ou prophylactiquement efficace du concentré de protéines du lactosérum.

17. Utilisation d'un concentré de protéines de lactosérum selon la revendication 15 pour la fabrication d'un médicament destiné à améliorer la résistance à l'acceptation chez les humains et chez les animaux d'une quantité thérapeutiquement ou prophylactiquement efficace de concentré de protéines de lactosérum.

18. Utilisation du concentré de protéines du lactosérum selon la revendication 17, dans lequel la résistance à l'acceptation améliorée comporte une résistance renforcée à l'infection bactérienne et/ou une résistance renforcée à la croissance lente de carcinomes et/ou une résistance renforcée au processus de vieillessement et/ou une combinaison de ce qui précède.

19. Utilisation d'un concentré de protéines de lactosérum selon la revendication 15, dans la fabrication d'une composition thérapeutique anti-cancer prévue pour la prophylaxie du cancer du côlon.
